# EUROPEAN PATENT SPECIFICATION

(11) **EP 4 610 366 B1**
(45) Date of publication and mention of the grant of the patent: **17.06.2026**
(21) Application number: 24160449.5
(22) Date of filing: 29.02.2024
(51) Int. Cl.: C12Q 1/6806

(54) **LENTIVIRUS INTEGRATION JUNCTION ANALYSIS**
VERBINDUNGSANALYSE ZUR INTEGRATION VON LENTIVIREN
ANALYSE DE JONCTION D'INTÉGRATION DE LENTIVIRUS

(43) Date of publication of application: 03.09.2025
(73) Proprietor: Miltenyi Biotec B.V. & Co. KG, 51429 Bergisch Gladbach (DE)
(72) Inventor: HOSONO, Seiyu, 51429 Bergisch Gladbach (DE); PINARD, Robert, 51429 Bergisch Gladbach (DE); MÜLLER, Reto, 51429 Bergisch Gladbach (DE); WANG, Jinling, 51429 Bergisch Gladbach (DE)
(74) Representative: Kisters, Michael Marcus

(56) References cited:
- WO-A1-2021/110878
- ALQUEZAR-PLANAS DAVID E. ET AL: "DNA sonication inverse PCR for genome scale analysis of uncharacterized flanking sequences", vol. 12, no. 1, 18 October 2020 (2020-10-18), pages 182 - 195, XP093145320, ISSN: 2041-210X, Retrieved from the Internet <URL:https://onlinelibrary.wiley.com/doi/full-xml/10.1111/2041-210X.13497> DOI: 10.1111/2041-210X.13497

## Description

### BACKGROUND

Lentiviral vector delivery has advantages over other gene-therapy methods due to high-efficiency of infection of both dividing and non-dividing cells, long-term stable expression of a transgene, and low immunogenicity. Lentiviruses have been used to elicit an immune response against tumor antigens. However, like most current gene therapy experiments, establishment of a quick and accurate method of quality assessment of the virus delivery and infection is necessary.

Quick and efficient analysis of the location and the lentivirus provirus integration in the host human genome is essential for assessing the quality of viral transduction used for gene therapy.

This invention describes the method for assessing the location and optionally the copy number of lentiviral integration in the host human genome based on shearing the extracted genomic DNA followed by inverse PCR using a primer set from lentiviral LTR (Long terminal repeat) region and sequencing by Rolling Circle Amplification. The sequencing result is then analyzed by a customized bioinformatic algorithm to fish out the exact integration junction between the lentivirus provirus and the host genome.

Similar methods to analyze DNA fragments are known from ALQUEZAR-PLANAS DAVID E. ET AL: "DNA sonication inverse PCR for genome scale analysis of uncharacterized flanking sequences", METHODE IN ECOLOGY AND EVOLUTION, vol. 12, no. 1, 18 October 2020 (2020-10-18), pages 182-195 and WO2021110878.

### SUMMARY

The goal of this invention is to provide a method to obtain the exact genomic integration location and optionally the copy number information of the transduced and integrated lentivirus (provirus) with a higher resolution than the known technologies.

The method involves PCR pre-amplifying a fragment of genomic DNA which carries the junction sequence of 5' and 3' LTR region and the host genomic DNA and rolling circle amplifying the PCR product.

### OBJECT OF THE INVENTION

Object of the invention is a method to obtain the genomic location of a provirus sequence embedded by two LTR regions in a DNA strand **characterized by** the steps
a. fragmentation of the DNA strand into a plurality of strands having a length of 50 to 1000 bp thereby obtaining a mixture of strands comprising at least one LTR region of the provirus sequence and a junction region of the DNA strand having 20 to 100 bp and strands not comprising an LTR region
b. Converting the strands into circularized strands
c. Providing PCR primers P1 and P2 to the 3' and 5' ends of the at least one LTR region in the circularized strands
d. Multiplying the circularized strands provided with the PCR primers by inverse PCR, thereby obtaining a plurality of linear strands wherein the at least one LTR region and the junction regions are embedded by the PCR primers
e. Converting the linear strands with embedded LTR and junction regions into circularized and amplification of the circularized strands by RCA into rolonies
f. Obtaining the sequence information of the rolonies, thereby obtaining the sequence information of the junction regions
g. Aligning the sequence information of the junction regions with the sequence information of the DNA strand, thereby obtaining the genomic location of the provirus sequence.

The method according to the invention is especially suitable to obtain the genomic location of a provirus sequence in a DNA strand which is the human genome sequence of the human chromosomes.

The provirus sequence may origin from any kind of virus like a lentivirus. The term "provirus sequence" and "lentivirus sequence" are used interchangeable.

In addition to obtain the genomic location of a provirus sequence, the number of copies of the provirus sequence in the DNA strand can be obtained.

To this end, the sequence information of the DNA strand should be obtained either before or after the method. Since the human genome sequence is known, this information is accessible to the person skilled in the art. However, the sequence information of the DNA strand can also be obtained by the standard DNA sequencing methods.

### BRIEF DESCRIPTION OF THE DRAWINGS

Fig. 1 shows the general workflow of the method of the invention
Fig. 2 shows circularized strands with PCR primers P1 and P2 provided to the at least one LTR region (Step c)
Fig. 3 shows the linear strands multiplied by PCR obtained in step d)
Fig. 4 shows the exact DNA sequence of the construct of PCR primer used in inverse PCR reaction described in Figure 2 and 3.
Fig. 5 and 6 show the bioinformatic workflow of aligning the information of the junction region with the sequence of the DNA strand (step g)
Fig. 7 shows the location of the lentiviral insertion site along the lentivirus genome. All reads mapped to 5' and 3' terminal region of the LTR region as expected in Lentivirus integrated samples.
Fig. 8 shows the unique insertion site distribution along host cell genome in an test experiment performed with Lentivirus integrated samples (GFP+).
Fig. 9 shows the summary of the proof-of-concept experiment from 2 different methods (long primer blunt ligase and short primer Circligase) on positive control GFP+ and negative control GFP- samples. IS merge is the total number of samples that was found to be integrated within certain genes.
Fig. 10 shows the detailed result (from 4 individual sequencing datasets) from 2 different methods (long primer blunt ligase and short primer Circligase) on positive control GFP+.
Fig. 11 shows how to determine the provirus (integrated lentivirus) copy number per cell.

### DETAILED DESCRIPTION

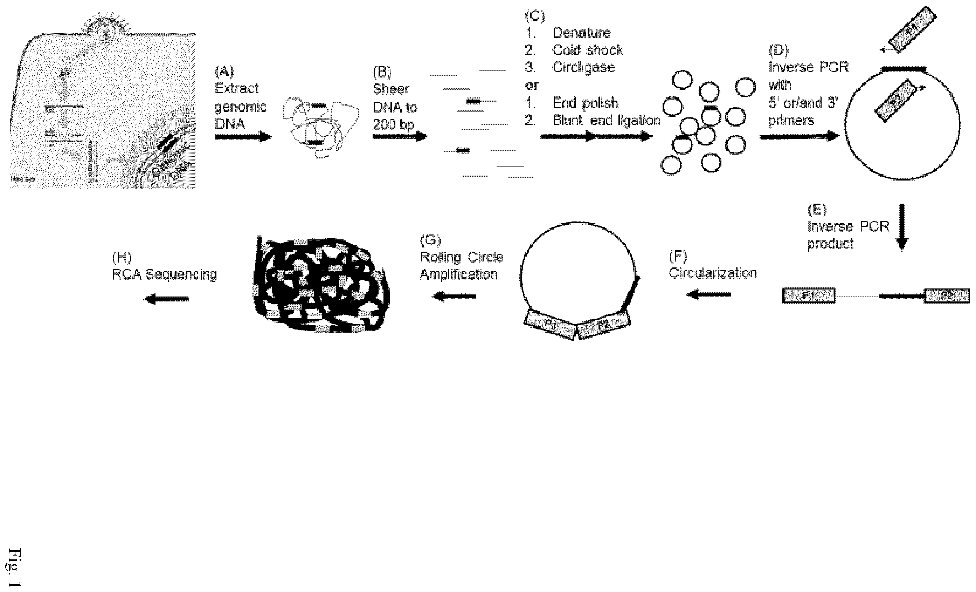

The general method of the invention is shown in Fig. 1 which is now described in detail. The method is employed at cells transduced with a lentivirus. Such transduction processes are known the person skilled in the art.

First, genomic DNA is extracted from transduced cells (Fig 1A).

Next, the genomic DNA is sheared either mechanically or enzymatically preferable to an average size of 100 - 300 base pairs (bp) like 200 bp. (Fig 1C).

In a first embodiment of the invention, the plurality of the strands is denatured into a plurality of single stranded DNA strands which is then converted into a plurality of circularized single stranded DNA. This can be achieved by mechanical denaturing, cold shocking and single strand DNA ligation (e.g. by Circligase) of the denatured single strand.

In a different approach, the sheared genomic DNA is end polished and blunt end ligated with T4 DNA Ligase (Fig 1C). Accordingly, in a second embodiment of the invention, after step a), the plurality of the strands is provided with blunt ends by a ligase to obtain a plurality of double stranded DNA strands which is then converted into a plurality of circularized double stranded DNA, preferable by a T4 ligase.

Next, the circularized genomic DNA which contains LTR sequence are amplified with Polymerase Chain Reaction using inverse PCR primers with adapters with a fixed DNA sequence P1 and P2 which binds to the LTR regions of Lentivirus (Fig 1D and 1E).

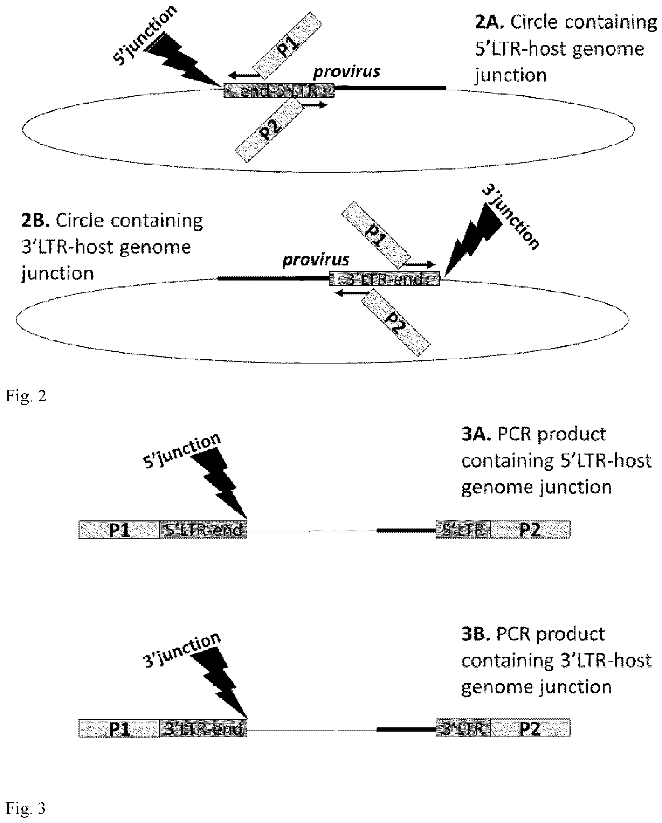

Two sets of PCR primers may be used. One set is specific for 5'LTR and another set is specific for 3'LTR since 5'LTR and 3'LTR will not coexist in the same sheared DNA molecule. The directionality of the PCR primer for 5'LTR region is so that primer P1 is facing towards the 5' end of the 5'LTR (Fig 2A).

In a third embodiment of the method, the circularized strands provided with the PCR primers comprises a mixture of first circularized strands having junction regions in 3' direction of the LTR region, and second circularized strands having junction regions in 5' direction of the LTR region, and wherein the first and second circularized strands are independently multiplied by inverse PCR thereby obtaining of linear strands having junction regions in 3' direction of the LTR region or linear strands having junction regions in 5' direction of the LTR region.

In a forth embodiment of the method, the PCR primers P1 and/or P2 are provided to the respective 3' and/or 5' ends of the at least one LTR region in the circularized strands such that PCR primer P1 is facing towards the 5' end of the 5'LTR or 3'LTR region and PCR primer P2 is facing towards the 3' end of the 5'LTR or 3'LTR region.

In this embodiment, the directionality of the PCR primer for 3'LTR region is so that primer P1 is facing towards the 3' end of the 3'LTR (Fig 2B). That way both genomic insertion junctions of the provirus can be mapped simultaneously. The orientation of the 5'LTR PCR product (Fig 3A) and 3'LTR PCR product (Fig 3B) is depicted.

Preferable, step e) is performed by using a DNA splint bridge oligonucleotide which brings the P1 and P2 ends together and ligation by T4 DNA Ligase (Fig 1F).

The circularized strand is then amplified by Rolling Circle Amplification (RCA) (Fig 1G) into so called rollonies. Rolonies comprise a plurality of linear concatamers of the circularized strand.

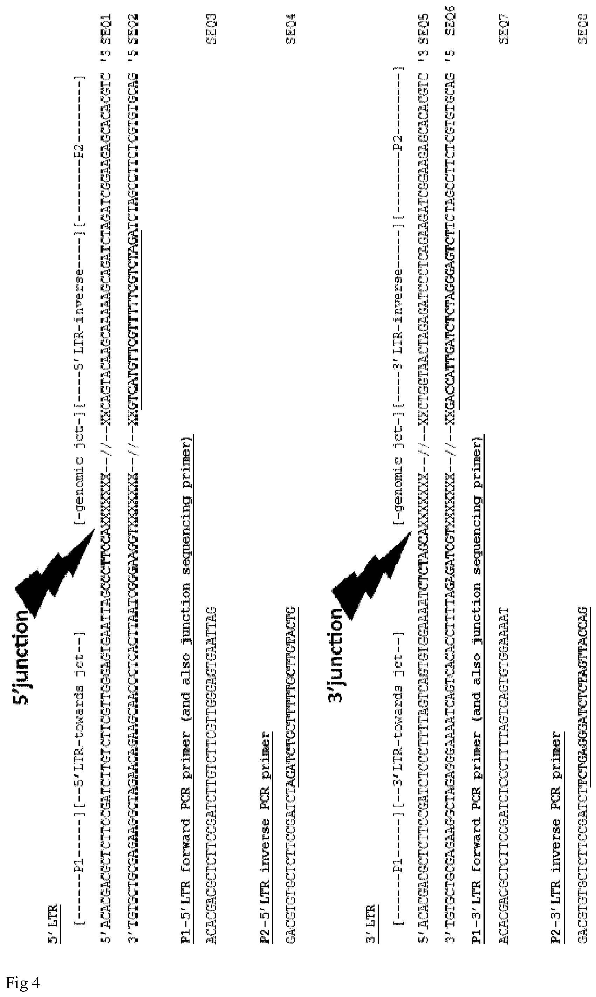

The resulting RCA product (rolony) is then NGS sequenced using a 5'LTR junction sequencing primer which binds 8 bases upstream of the 5' end of LTR and the genomic junction, and a 3'LTR junction sequencing primer which binds 8 bases upstream of the 3' end of LTR and the genomic junction so that one can distinguish between the two ends (Fig 4).
By NGS sequencing of the LTR and genomic DNA junction can be analyzed using the analysis workflow described below.

Step g) of the method of the invention may be performed by mapping the sequence information of the junction regions to the sequence information of the DNA strand wherein regions of the DNA strand aligned with high confidence (MAPQ >=30) with to the sequence information of the junction regions are designated as genomic location of the provirus sequence. Aligning may include merging overlapping sites, and annotation with intersecting genes, or the closest genes if no intersecting genes were identified.

### EXAMPLE

This example describes the method for assessing the location and copy number of lentiviral integrations in the host human genome according to the invention.

The method involves PCR pre-amplifying a fragment of genomic DNA which carries the junction sequence of 5' and 3' LTR region and the host genomic DNA and rolling circle amplifying the PCR product.

### Transduction/multiplication steps

The genomic DNA from a Lentivirus vector transduced SUP-T1 cells (Human T lymphoblast cell line from ATCC) is extracted and used against a genomic DNA extracted from a non-transduced cells as negative control. By way of example, a lentiviral vector construct derived from HIV was used, which is a highly efficient vehicles for in vivo gene delivery. The construct carries a Green Fluorescence Protein (GFP) which exhibits green fluorescence when exposed to light in the blue to ultraviolet range which serves as a marker for positive transduction and integration of provirus into the host genome.

First, genomic DNA extracted from lentivirus transduced (GFP+ plus) and non-transduced (GFP- minus) SUP-T1 cells (Fig 1A).

Next, the genomic DNA samples were sheared mechanically by sonication (Covaris Ultrasonicator) by setting the desired size to 200 base pair (Fig 1C). The concentration of sheared DNA was 200 nanogram in 130uL volume of 1XTris/EDTA (1.54 ng/uL).

Next, in one method, 50 nanogram of sheared genomic DNA was denatured at 95°C for 10 min, cold shocked at 4°C and was set on ice. Ligation of a single-stranded circle was performed with Circligase at 60°C for 60 min and inactivated at 80°C for 10 min. The ligation reaction was cleaned up to remove uncircularized molecule by incubate with Exonuclease I and III at 37°C for 60 min and purified with Spry Bead or size exclusion column.

Alternatively, the fragmented genomic DNA was end polished and blunt-end ligated with T4 DNA Ligase (Fig 1C).

Next the circularized genomic DNA which contains LTR sequence is amplified with Polymerase Chain Reaction using inverse PCR primers with adapters with a fixed DNA sequence P1 and P2 which binds to the LTR regions of Lentivirus (Fig 1D and 1E).

Two sets of PCR primers are used. One set is specific for 5'LTR and another set is specific for 3'LTR since 5'LTR and 3'LTR will not coexist in the same sheared DNA molecule of 200 base pair size. The directionality of the PCR primer for 5'LTR region is so that primer P1 is facing towards the 5' end of the 5'LTR (Fig 2A).

In contrast, the directionality of the PCR primer for 3'LTR region is so that primer P1 is facing towards the 3' end of the 3'LTR (Fig 2B). That way both junctions of the provirus can be mapped simultaneously. The orientation of the 5'LTR PCR product (Fig 3A) and 3'LTR PCR product (Fig 3B) is depicted.

The PCR amplified product can then be circularized using the splint bridge oligonucleotide primer which brings the P1 and P2 ends together (Fig 1F).

The circle is then amplified by Rolling Circle Amplification (RCA) (Fig 1G).

The resulting RCA product is then NGS sequenced using a 5'LTR junction sequencing primer which binds 8 bases upstream of the 5' end of LTR and the genomic junction, and a 3'LTR junction sequencing primer which binds 8 bases upstream of the 3' end of LTR and the genomic junction so that one can distinguish between the 3' and 5' ends (Fig 4).

The genomic DNA/LTR junctions can then be sequenced, and the junction region can then be defined against the genomic DNA reference sequence.

### Bioinformatic insertion site analysis workflow is performed to determine integrated sites (coordinates on the chromosome).

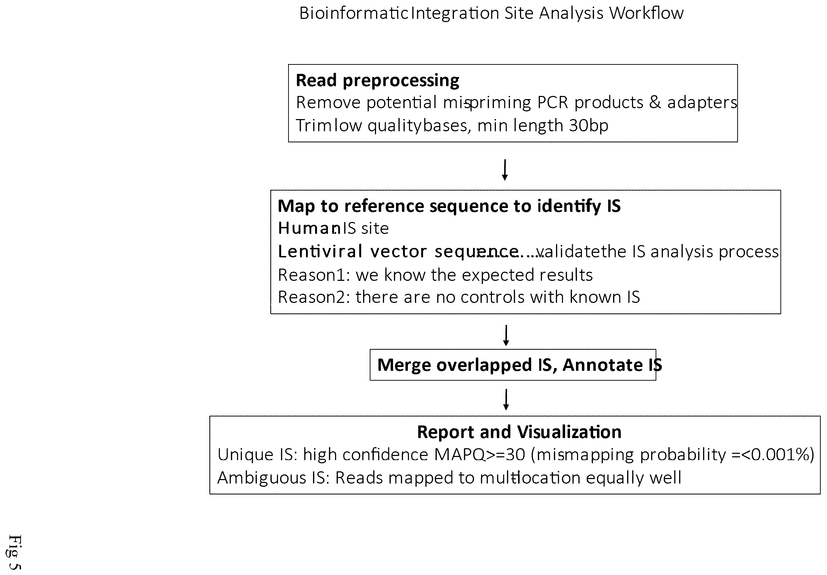

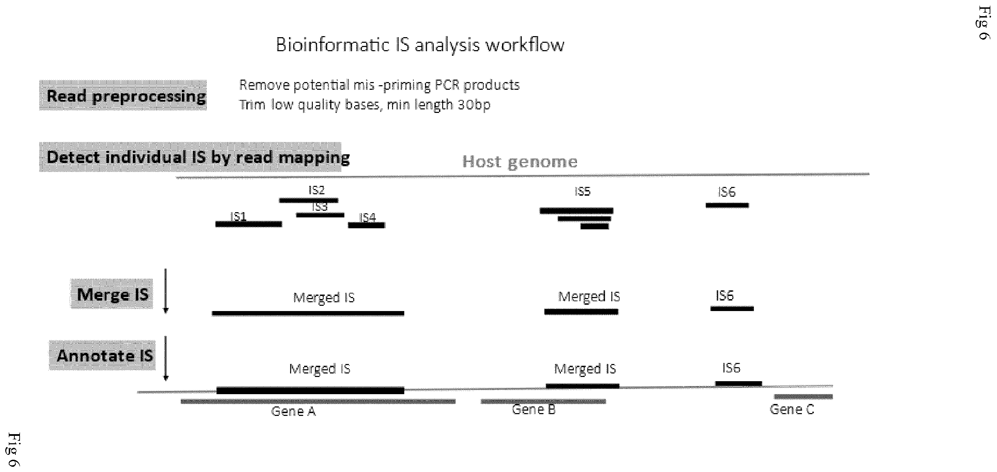

Figure 5 and 6 show the bioinformatic workflow for analyzing the sequencing result of the integration site (IS).

Read preprocessing is performed by removing potential mis-priming of PCR products & adapters. Low quality bases are trimmed, min length 30 bases pairs.

Next, the sequence is mapped to human reference sequence to identify the IS.

The sequence is also mapped to lentiviral vector sequence in order to validate the IS analysis process.

Next overlapped IS are merged and IS are annotated.

At the end, a report is generated and visualized.

Unique IS are identified with high confidence MAP Q>=30 (mis-mapping probability =<0.001%).

Ambiguous IS are also identified (Reads mapped to multi-location equally well).

Figure 6 shows the bioinformatic workflow for analyzing the sequencing result of the integration site (IS).

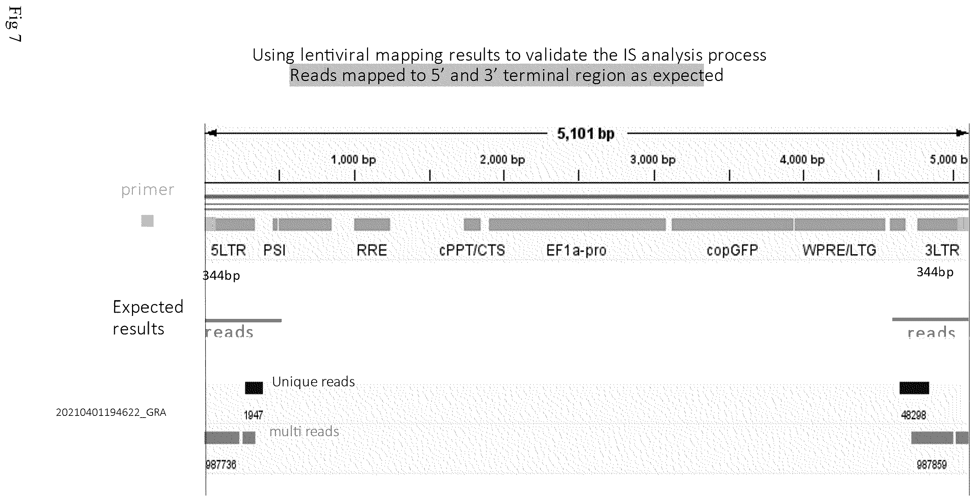

Figure 7 to 10 shows a sample result generated from a proof-of -concept experiment performed with Lentivirus integrated samples (GFP+ plus) and a negative control (GFP- minus).

Figure 7 shows that all the lentiviral insertion site mapping resulted in reads mapped to 5' and 3' terminal region of the LTR region as expected in Lentivirus integrated samples (GFP+). The number of read in 3' and 5' end was very close to each other (987,736 vs 987,859) which shows that this method is performing on both 3' and 5' ends equally well. The fact that the reads only mapped to LTR and adjacent regions, but not virus internal region suggests that this method is performing well.

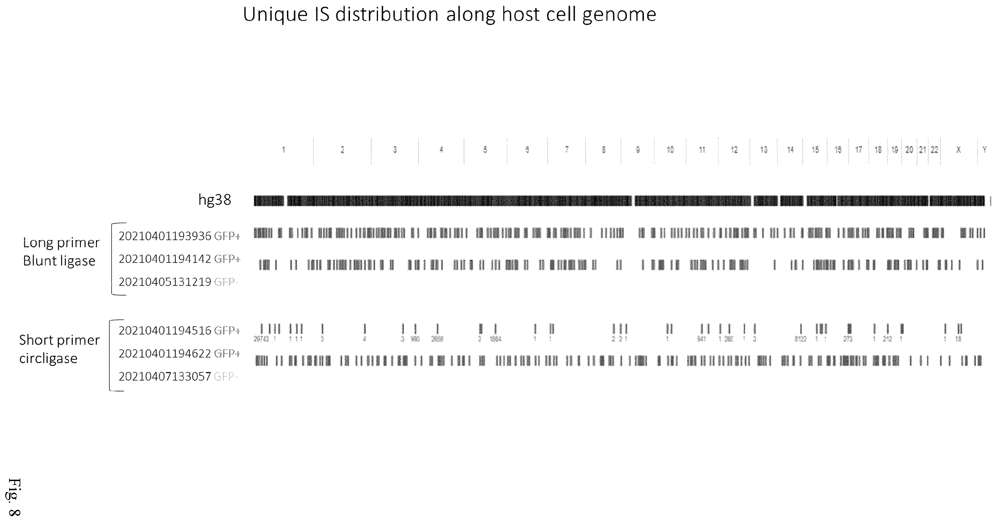

Figure 8 shows unique insertion site distribution along host cell genome in Lentivirus integrated samples (GFP+). Long primer blunt ligation method and short primer circligase method both showed broad distribution along the host chromosome in different GFP+ samples.

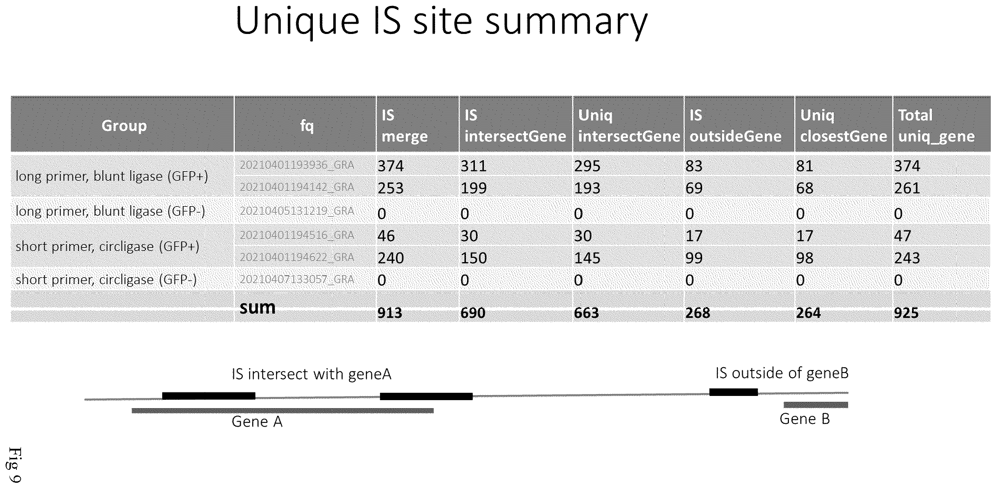

Figure 9 shows the summary of the proof-of-concept experiment from 2 different methods (long primer blunt ligase and short primer circligase) on positive control GFP+ and negative control GFP- samples. IS merge is the total number of samples that was found to be integrated within certain genes. IS intersectGene is the total number of samples that was found to intersect certain genes. Uniq intersectGene is the total number total number of unique genes that intersect with viral insertion site. IS outsideGene is the total number of samples that was found outside of any genes. Uniq closestGene is the total number of unique genes that are close to 'IS outsideGene'. Total uniq-gene is the total number of genes that intersect or closest to IS (viral insertion site).

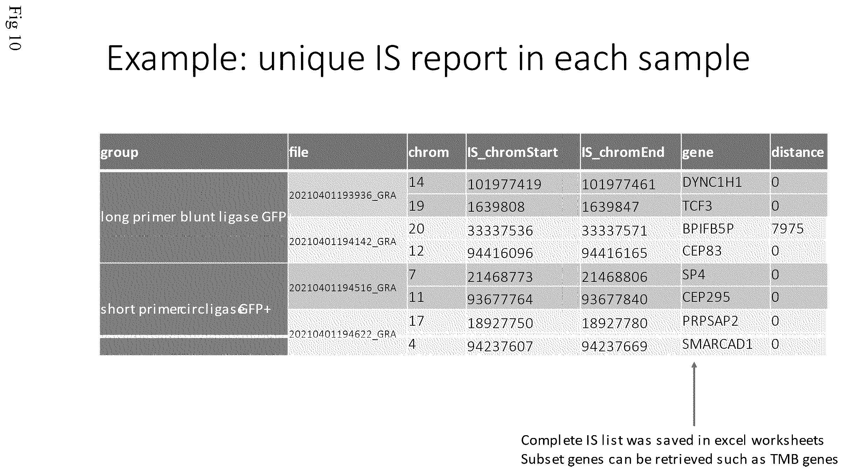

Figure 10 shows the detailed result (from 4 individual sequencing datasets) from 2 different methods (long primer blunt ligase and short primer circligase) on positive control GFP+. It shows in which chromosome the lentivirus was integrated (chrom). It shows where exactly the sequencing reads are mapped (IS_chromStart and IS_chromEnd) on which genes. It also shows the distance between the integration site and a certain gene.

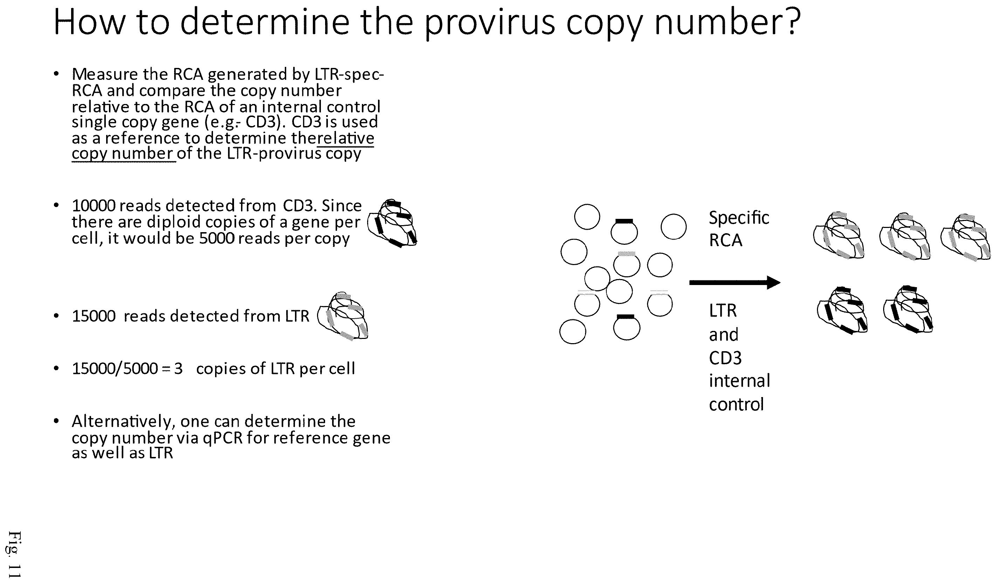

Figure 11 shows how to determine the provirus copy number. Use a single copy internal control gene e.g.- CD3 as a reference to determine the relative copy number of the LTR-provirus copy. First, RCAs are generated from LTR specific iPCR and single copy gene specific (e.g.- CD3) RCA. Secondly, NGS sequencing is performed on both LTR region and CD3 gene and read number is determined. Since single copy gene (CD3) has 2 copies per cell (diploid), divide the totally ready by 2 (e.g.- 10,000 read divide by 2 is 5,000 copies). Divide the number of reads from LTR by single copy gene CD3 (15,000/5,000 = 3). There are 3 copies of provirus per cell. Alternatively, one can determine the copy number using a quantitative PCR for reference gene as well as LTR region of provirus.

## Claims

1. A method to obtain the genomic location of a provirus sequence embedded by two LTR regions in a DNA strand **characterized by** the steps
a. fragmentation of the DNA strand into a plurality of strands having a length of 50 to 1000 bp thereby obtaining a mixture of strands comprising at least one LTR region of the provirus sequence and a junction region of the DNA strand having 20 to 100 bp and strands not comprising an LTR region
b. Converting the strands into circularized strands
c. Providing PCR primers P1 and P2 to the 3' and 5' ends of the at least one LTR region in the circularized strands
d. Multiplying the circularized strands provided with the PCR primers by inverse PCR, thereby obtaining a plurality of linear strands wherein the at least one LTR region and the junction regions are embedded by the PCR primers
e. Converting the linear strands with embedded LTR and junction regions into circularized and amplification of the circularized strands by RCA into rolonies
f. Obtaining the sequence information of the rolonies, thereby obtaining the sequence information of the junction regions
g. Aligning the sequence information of the junction regions with the sequence information of the DNA strand, thereby obtaining the genomic location of the provirus sequence.

2. Method according to claim 1 **characterized in that** after step a), the plurality of the strands is denatured into a plurality of single stranded DNA strands which is then converted into a plurality of circularized single stranded DNA.

3. Method according to claim 1 or 2 **characterized in that** after step a), the plurality of the strands is provided with blunt ends by a ligase to obtain a plurality of double stranded DNA strands which is then converted into a plurality of circularized double stranded DNA.

4. Method according to any of claims 1 to 3 **characterized in that** step e) is performed by using a DNA splint bridge oligonucleotide which brings the P1 and P2 ends together and ligation by T4 DNA Ligase.

5. Method according to any of claims 1 to 4 **characterized in that** the circularized strands provided with the PCR primers comprises a mixture of first circularized strands having junction regions in 3' direction of the LTR region, and second circularized strands having junction regions in 5' direction of the LTR region, and wherein the first and second circularized strands are independently multiplied by inverse PCR thereby obtaining of linear strands having junction regions in 3' direction of the LTR region or linear strands having junction regions in 5' direction of the LTR region.

6. Method according to any of claims 1 to 5 **characterized in that** the PCR primers P1 and/or P2 are provided to the respective 3' and/or 5' ends of the at least one LTR region in the circularized strands such that PCR primer P1 is facing towards the 5' end of the 5'LTR or 3'LTR region and PCR primer P2 is facing towards the 3' end of the 5'LTR or 3'LTR region.

7. Method according to any of claims 1 to 6 **characterized in that** the number of copies of the provirus sequence in the DNA strand is obtained.

8. Method according to any of claims 1 to 7 **characterized in** obtaining the sequence information of the DNA strand.

9. Method according to any of claims 1 to 8 **characterized in that** step g) is performed by mapping the sequence information of the junction regions to the sequence information of the DNA strand wherein regions of the DNA strand aligned with high confidence (MAPQ >=30) with to the sequence information of the junction regions are designated as genomic location of the provirus sequence.

## Patentansprüche

1. Verfahren zum Erhalten der genomischen Position einer Provirus-Sequenz, die in zwei LTR-Regionen in einem DNA-Strang eingebettet ist, **gekennzeichnet durch** die folgenden Schritte
a. Fragmentieren des DNA-Strangs in eine Vielzahl von Strängen mit einer Länge von 50 bis 1.000 bp, wodurch ein Gemisch aus Strängen erhalten wird, das mindestens eine LTR-Region der Provirus-Sequenz und eine Verbindungsregion des DNA-Strangs mit einer Länge von 20 bis 100 bp umfasst, sowie Stränge, die keine LTR-Region umfassen,
b. Umwandeln der Stränge in zirkularisierte Stränge,
c. Bereitstellen der PCR-Primer P1 und P2 an den 3'- und 5'-Enden der mindestens einen LTR-Region in den zirkularisierten Strängen,
d. Vervielfältigen der zirkularisierten Stränge, an denen die PCR-Primer bereitgestellt wurden, durch inverse PCR, wodurch eine Vielzahl linearer Stränge erhalten wird, in denen die mindestens eine LTR-Region und die Verbindungsregionen in die PCR-Primer eingebettet sind
e. Umwandeln der linearen Stränge mit eingebetteten LTR- und Verbindungsregionen in zirkularisierte Stränge und Amplifizieren der zirkularisierten Stränge mittels RCA zu Rolonies,
f. Erhalten der Sequenzinformationen der Rolonies, wodurch die Sequenzinformationen der Verbindungsregionen erhalten werden,
g. Alignieren der Sequenzinformationen der Verbindungsregionen mit den Sequenzinformationen des DNA-Strangs, wodurch die genomische Position der Provirus-Sequenz erhalten wird.

2. Verfahren nach Anspruch 1, **dadurch gekennzeichnet, dass** nach Schritt a) die Vielzahl der Stränge in eine Vielzahl von einzelsträngigen DNA-Strängen denaturiert wird, die dann in eine Vielzahl von zirkularisierten einzelsträngigen DNA-Strängen umgewandelt wird.

3. Verfahren nach Anspruch 1 oder 2, **dadurch gekennzeichnet, dass** nach Schritt a) an der Vielzahl der Stränge durch eine Ligase stumpfe Enden bereitgestellt werden, um eine Vielzahl von doppelsträngigen DNA-Strängen zu erhalten, die dann in eine Vielzahl von zirkularisierten doppelsträngigen DNA-Strängen umgewandelt wird.

4. Verfahren nach einem der Ansprüche 1 bis 3, **dadurch gekennzeichnet, dass** Schritt e) unter Verwendung eines DNA-Brückenoligonukleotids durchgeführt wird, das die P1- und P2-Enden zusammenführt, und durch Ligierung mittels T4-DNA-Ligase.

5. Verfahren nach einem der Ansprüche 1 bis 4, **dadurch gekennzeichnet, dass** die zirkularisierten Stränge, an denen die PCR-Primer bereitgestellt wurden, ein Gemisch aus ersten zirkularisierten Strängen mit Verbindungsregionen in 3'-Richtung der LTR-Region und zweiten zirkularisierten Strängen mit Verbindungsregionen in 5'-Richtung der LTR-Region umfassen, und wobei die ersten und zweiten zirkularisierten Stränge unabhängig voneinander durch inverse PCR vervielfältigt werden, wodurch lineare Stränge mit Verbindungsregionen in 3'-Richtung der LTR-Region oder lineare Stränge mit Verbindungsregionen in 5'-Richtung der LTR-Region erhalten werden.

6. Verfahren nach einem der Ansprüche 1 bis 5, **dadurch gekennzeichnet, dass** die PCR-Primer P1 und/oder P2 an den jeweiligen 3'- und/oder 5'-Enden der mindestens einen LTR-Region in den zirkularisierten Strängen bereitgestellt werden, derart, dass der PCR-Primer P1 zum 5'-Ende der 5'LTR- oder 3'LTR-Region hin ausgerichtet ist und der PCR-Primer P2 zum 3'-Ende der 5'LTR- oder 3'LTR-Region hin ausgerichtet ist.

7. Verfahren nach einem der Ansprüche 1 bis 6, **dadurch gekennzeichnet, dass** die Anzahl der Kopien der Provirus-Sequenz im DNA-Strang erhalten wird.

8. Verfahren nach einem der Ansprüche 1 bis 7, **dadurch gekennzeichnet, dass** die Sequenzinformationen des DNA-Strangs erhalten werden.

9. Verfahren nach einem der Ansprüche 1 bis 8, **dadurch gekennzeichnet, dass** Schritt g) durchgeführt wird, indem die Sequenzinformationen der Verbindungsregionen auf die Sequenzinformationen des DNA-Strangs gemappt werden, wobei Regionen des DNA-Strangs, die mit hoher Zuverlässigkeit (MAPQ >=30) mit den Sequenzinformationen der Verbindungsregionen aligniert sind, als genomische Position der Provirus-Sequenz ausgewiesen werden.

## Revendications

1. Procédé permettant d'obtenir la localisation génomique d'une séquence provirale encadrée par deux régions LTR dans un brin d'ADN **caractérisé par** les étapes
a. fragmentation du brin d'ADN en une pluralité de brins ayant une longueur de 50 à 1000 pb obtenant ainsi un mélange de brins comprenant au moins une région LTR de la séquence provirale et une région de jonction du brin d'ADN ayant de 20 à 100 pb et de brins ne comprenant pas de région LTR
b. conversion des brins en brins circularisés
c. fourniture d'amorces PCR P1 et P2 aux extrémités 3' et 5' de l'au moins une région LTR dans les brins circularisés
d. multiplication des brins circularisés fournis avec les amorces PCR par PCR inverse obtenant ainsi une pluralité de brins linéaires dans lesquels l'au moins une région LTR et les régions de jonction sont encadrées par les amorces PCR
e. conversion des brins linéaires avec des régions LTR et de jonction encadrées en brins circularisés et amplification des brins circularisés par RCA en colonies
f. obtention des informations de séquence des colonies, obtenant ainsi les informations de séquence des régions de jonction
g. alignement des informations de séquence des régions de jonction avec les informations de séquence du brin d'ADN, obtenant ainsi la localisation génomique de la séquence provirale.

2. Procédé selon la revendication 1 **caractérisé en ce qu'**après l'étape a), la pluralité des brins est dénaturée en une pluralité de brins d'ADN simple brin qui est ensuite convertie en une pluralité d'ADN simple brin circularisés.

3. Procédé selon la revendication 1 ou 2 **caractérisé en ce qu'**après l'étape a), la pluralité de brins est fournie avec des extrémités franches par une ligase afin d'obtenir une pluralité de brins d'ADN double brin qui est ensuite convertie en une pluralité d'ADN double brin circularisés.

4. Procédé selon l'une quelconque des revendications 1 à 3 **caractérisé en ce que** l'étape e) est réalisée à l'aide d'un oligonucléotide pont d'épissage d'ADN qui réunit les extrémités P1 et P2 et une ligature par la T4 ADN ligase.

5. Procédé selon l'une quelconque des revendications 1 à 4 **caractérisé en ce que** les brins circularisés fournis avec les amorces PCR comprennent un mélange de premiers brins circularisés ayant des régions de jonction en direction 3' de la région LTR, et de seconds brins circularisés ayant des régions de jonction en direction 5' de la région LTR, et dans lequel les premiers et seconds brins circularisés sont multipliés indépendamment par PCR inverse obtenant ainsi des brins linéaires ayant des régions de jonction en direction 3' de la région LTR ou des brins linéaires ayant des régions de jonction en direction 5' de la région LTR.

6. Procédé selon l'une quelconque des revendications 1 à 5 **caractérisé en ce que** les amorces PCR P1 et/ou P2 sont fournies aux extrémités 3' et/ou 5' respectives de l'au moins une région LTR dans les brins circularisés de telle sorte que l'amorce PCR P1 est orientée vers l'extrémité 5' de la région 5'LTR ou 3'LTR et que l'amorce PCR P2 est orientée vers l'extrémité 3' de la région 5'LTR ou 3'LTR.

7. Procédé selon l'une quelconque des revendications 1 à 6 **caractérisé en ce que** le nombre de copies de la séquence provirale dans le brin d'ADN est obtenu.

8. Procédé selon l'une quelconque des revendications 1 à 7 **caractérisé dans** l'obtention des informations de séquence du brin d'ADN.

9. Procédé selon l'une quelconque des revendications 1 à 8 **caractérisé en ce que** l'étape g) est réalisée en mappant les informations de séquence des régions de jonction sur les informations de séquence du brin d'ADN dans lequel les régions du brin d'ADN alignées avec une confiance élevée (MAPQ >=30) avec les informations de séquence des régions de jonction sont désignées comme la localisation génomique de la séquence provirale.
